**Europäisches Patentamt**

**European Patent Office**

**Office européen des brevets**

(19)

(11) Veröffentlichungsnummer : **0 117 888 B2**

(12) # NEUE EUROPÄISCHE PATENTSCHRIFT

(45) Veröffentlichungstag der neuen Patentschrift :
**26.08.92 Patentblatt 92/35**

(51) Int. Cl.$^5$ : **A61K 31/44, A61K 9/08**

(21) Anmeldenummer : **83102475.7**

(22) Anmeldetag : **14.03.83**

(54) **Flüssigzubereitungen von Dihydropyridinen, ein Verfahren zu ihrer Herstellung, sowie ihre Verwendung bei der Bekämpfung von Erkrankungen.**

(30) Priorität : **03.03.83 DE 3307422**

(43) Veröffentlichungstag der Anmeldung :
**12.09.84 Patentblatt 84/37**

(45) Bekanntmachung des Hinweises auf die Patenterteilung :
**16.09.87 Patentblatt 87/38**

(45) Bekanntmachung des Hinweises auf die Entscheidung über den Einspruch :
**26.08.92 Patentblatt 92/35**

(84) Benannte Vertragsstaaten :
**AT BE CH DE FR GB IT LI LU NL SE**

(56) Entgegenhaltungen :
**EP-A- 49 852**
**EP-A- 0 004 650**
**GB-A- 817 623**
**GB-A- 2 084 017**

(56) Entgegenhaltungen :
**CHEMICAL ABSTRACTS, Band 91, Nr. 18, 29. Oktober 1979, Seite 331, Nr. 145969m, Columbus, Ohio,USA**
**CHEMICAL ABSTRACTS, Band 91, Nr. 20, 12. November 1979, Seite 350, Nr. 163057s, Columbus, Ohio,USA**
**Inaugural-Dissertation von Reinhardt Klimek, Frankfurt am Main, 1978, S. 35-39**
**Angewandte Biopharmazie von W.A. Ritschel, Wissensch. Verlagsges. m. b. H.,1973, S. 281-288**
**Arzneikapseln von Czetsch-Lindenwald u. W.Fahrig, 1962, Editio Cantor/Aulendorf S.34**
**Journal of Pharm. Soc. Japan, Bd.73,1953, S. 469-475 Sci.Pharm.33(1965)2,S.90-101**

(73) Patentinhaber : **BAYER AG**
**W-5090 Leverkusen 1 Bayerwerk (DE)**

(72) Erfinder : **Hoff, Dieter, Dr.**
**Edmund-Husserl-Strasse 19**
**W-5090 Leverkusen 3 (DE)**
Erfinder : **Rämsch, Klaus-Dieter, Dr.**
**Scheidt-Strasse 141**
**W-5600 Wuppertal 21 (DE)**

EP 0 117 888 B2

## Beschreibung

Die Erfindung betrifft Flüsigzubereitungen von Nimodipin, insbesondere Tropfenformulierungen, ein Verfahren zu ihrer Herstellung, sowie ihre Verwendung bei der Bekämpfung von Erkrankungen.

Es ist bekannt, dass Dihydropyridine sehr starke kreislaufbeeinflussende Wirkungen besitzen (vgl. britisches Patent 1 173 862). Aufgrund ihrer Lichtempfindlichkeit und ihrer schweren Löslichkeit treten bei der galenischen Zubereitung von Arzneispezialitäten eine Reihe von Schwierigkeiten auf, wie auf zahlreichen Patenten und Patentanmeldungen für spezielle Formulierungen dieses Wirkstoffs ersichtlich wird. So betriff z.B. das US-Patent 3 784 684 spezielle Nifedipin enthaltende Gelatinebeisskapseln, durch welche die Coronarwirkung von Nifedipin vorteilhaft genutzt werden kann. In dem britischen Patent 1 456 618 werden weiterhin feste Arzneizubereitung beschrieben, welche ebenfalls eine gute Bioverfügbarkeit von Nifedipin gewährleiste. Auch in der DE-OS 2 822 882 werden feste Arzneiformen beschrieben, bei denen durch den Einsatz bestimmter Lösungsvermittler und oberflächenaktiver Substanzen die Schwerlöslichkeit von Nifedipin kompensiert werden soll. Auch in der europäischen Offenlegungsschrift 1 247 soll die Resorbierbarkeit von Nifedipin durch die Verwendung von Polyethylenglykol und bestimmter poröser Trägersubstanzen verbesser werden. In der Dissertation von R.Klimek, Frankfurt/Main 1978, "Untersuchungen zur Stabilitäts Kinetik photoinstabiler Arzneistoffe," S.35-39, geht es im wesentlichen um die Untersuchung der Lichtstabilität von Nifedipin durch den Zusatz des Tensids Tween 20. Insbesondere für den speziellen Wirkstoff Nimodipin, dessen Wasserlöslichkeit noctural um den Faktor 4 geringer ist als für Nifedipin, enthält diese Entgegenhaltung jedoch keinen Hierweis.

Aus EP-A 4 650 sind flüssige Zubereitungen von Nimodipin bekannt mit einem niedrigen Wirkstoffgehalt. Versucht man nach der dort gegebenen Lehre den Wirkstoffgehalt zu erhöhen, so tritt eine Rekristallisation ein, was zu einer Verschlechterung der Bioverfügbarkeit führt. Auch in der GB-A 2 084 017 wird ein Lösungsmittelgemisch beschrieben welches Dihydropyridine zu lösen vermag. Aus diesen Lösungen fällt jedoch der Wirkstoff bei Verdünnung mit wässrigen Medien direkt wieder aus. Damit lassen sich diese Lösungen nicht mit Wasser verdünnen bzw. zeigen eine Rekristallisation auch nach direkter Einnahme im Magen-Darm-Trakt und stehen somit nicht mehr in gelöster Form zur optimalen Resorption zur Verfügung. Das gleiche gilt für die Publikation in Chem. Abstr. 91 (1979) Ref. Nr. 145 969m. Auch in Chem. Abstr. 91 (1979) Ref. Nr. 163 057s werden ölige Lösungen beschrieben, die nicht wasserverdünnbar sind und auch geschmacklich für den Patienten nicht zumutbar sind.

Alle bisherigen Versuche, die schlechte Löslichkeit von Nifedipin durch bestimmte Massnahmen zu kompensieren und gleichzeitig eine gute Bioverfügbarkeit zu gewährleisten, besitzen eine Reihe von Nachteilen. Der Einsatz von oberflächenaktiven Substanzen, Lösungsvermittlern und bestimmten Trägerstoffen, die eine besondere Oberfläche haben, z.B. porös sind, führt häufig zu Verabreichungsformen bei denen die Präparate unerwünscht gross sind. Zur Erleichterung des Schluckens werden solche Tabletten oder Kapseln häufig in spezifische Formen wie z.B. Elipsoide oder Längsformen überführt, was jedoch bei Präparaten mit einem Gewicht über 400 mg auch nicht mehr zu befriedigenden Ergebnissen führt. Auch das häufigere Einnehmen von kleineren Präparaten stellt keine befriedigende Lösung dar.

Ebenfalls bekannt ist eine Nifedipin enthaltende Tablette, die dadurch gekennzeichnet ist, dass der kristalline Wirkstoff eine bestimmte spezifische Oberfläche aufweist (DE-OS 3 033 919). Nach oraler Applikation der Tablette steigt die Plasmakonzentration an und hält sich für viele Stunden auf einem hohen Wert.

Es besteht nach wie vor ein Bedürfnis, für Dihydropyridine eine Formulierung bereitzustellen, die eine sehr schnelle Wirkstoffresorption aufweist. Bei der Applikation des Wirkstoffs als Tablette steigt die Plasmakonzentration aufgrund der Schwerlöslichkeit nur langsam an. Demzufolge ist ein schneller Wirkungseintritt nicht zu erzielen.

Die Applikation des Wirkstoffes in Kapselform, in deren Kern der Wirkstoff gelöst ist, führt gegenüber der Darreichungsform einer Tablette zu einem schnelleren Aufbau der Plasmakonzentration, jedoch ist in vielen Fällen ein noch schnellerer Wirkungseintritt wünschenswert.

Darüber hinaus besteht ein Bedürfnis in der Medizin auch Tropfenformulierungen bereitzustellen, da vor allen Dingen ältere Patienten leichter zur Einnahme von Tropfen zu bewegen sind, als von Kapseln oder Tabletten.

Weiterhin können Tropfenformulierungen auch bewusstiosen Patienten ohne Komplikationen eingegeben werden. Ebenfalls kann der Wirkstoff durch Infusion dem Körper zugeführt werden.

Wie bereits eingangs geschildert, sind alle Dihydropyridine im wässrigen Medium schwer löslich. Beispielsweise beträgt für Nimodipin die Löslichkeit in Wasser 0.2 mg pro 100 ml.

Es wurde gefunden, dass bei Zugabe bestimmter Lösungsvermittler zu der Tropflösung in einer Konzentration von 30-50 Gewichtsprozent bezogen auf die Gesamtlösungsmenge, sich der sehr schwerlösliche Wirkstoff gut löst und auch in Lösung bleibt. Darüberhinaus wurde gefunden, dass bei einer Verdünnung der erfindungsgemässen Tropflösung mit wässrigen Medien eine solche Verdünnung je nach Verdünnungsgrad über einen längeren Zeitraum stabil und klar bleibt.

Die Erfindung betrifft somit Flüssigzubereitungen von Nimodipin der die dadurch gekennzeichnet sind, dass sie aus Nimodipin in Mengen von 1 bis 5 Gewichtsprozent, bezogen auf 100 Gewichtsteile einer Lösung bestehend aus 30-50 Gewichtsprozent, des Lösungsvermittlers Glycerin-Polyethylenglykoloxystearat mit ca. 35 Mol Ethylenoxyd, oder Glycerin-Polyethylenglykoloxystearat mit ca. 45 Mol Ethylenoxid und 70-50 Gewichtsprozent Ethanol, und gegebenenfalls weiteren Hilfsstoffen bestehen.

Zusätzlich können die Tropfen aromatisiert sein. In Betracht kommen beispielsweise:

Pfefferminzöl, Contramarum-Aroma, Zimt-Aroma Boonekamp-Aroma, Orangen-Aroma oder Citronen-Aroma.

Süssmittel, wie beispielsweise Saccharin oder Saccharin-Natrium können ebenfalls zugefügt werden.

Da Nimodipin sehr lichtempfindlich sind, kann es sich als notwendig erweisen dass den Tropflösungen als Lichtschutz und zwecks Stabilisierung bestimmte Farbstoffe zugesetzt werden müssen. Geeignete Farbstoffe können beispielsweise Apocarotenal, Canthaxanthin, Tartrazin (E 102), Amaranth (E 123), Erythrosin (E 127), insbesondere jedoch Gelborange S (E 110) sein. Die Konzentration des jeweiligen Farbstoffes beträgt 0,01-0,5 Gew.-%, vorzugsweise 0,1 -0,4 Gew.-%, bezogen auf die Flüssigzubereitung.

Die Herstellung der Flüssigkeitszubereitungen kann dadurch geschehen, dass man den Lösungsvermittler erwärmt, beispielsweise auf Temperaturen von 30 bis 70°C, das Nimodipin, beispielswveise unter Rühren, darin löst, das Verdünnungsmittel hinzufügt und gegebenenfalls die übrigen Bestandteile zugibt.

Beispiel 1

| | |
|---|---|
| Nimodipin | 40,000 g |
| Glycerin-Polyethylenglykol-oxystearat mit ca. 45 Mol Ethylenoxid | 400,000 g |
| Ethanol | 500,600 g |

Beispiel 2

| | |
|---|---|
| Nimodipin | 40,000 g |
| Glycerin-Polyethylenglykol-oxystearat mit ca. 45 Mol Ethylenoxid | 400,000 g |
| Contramarum-Aroma | 0,750 g |
| Ethanol | 500,600 g |

Beispiel 3

| | |
|---|---|
| Nimodipin | 40,000 g |
| Glycerin-Polyethylenglykol-oxystearat mit ca. 35 Mol Ethylenoxid | 435,000 g |
| Wasser entmineralisiert | 50,000 g |
| Saccharin Natrium | 9,000 g |
| Zimt-Aroma | 0,500 g |
| Ethanol | 529,200 g |

Die Flüssigzubereitung eignet sich aufgrud ihrer Eigenschaften zur Behandlung von cerebralen Durchblutungsstörungen.

Im allgemeinen hat es sich als vorteilhaft erwiesen, bei intravenöser Applikation Mengen von etwa 0.001 bis 10 mg/kg, vorzugsweise etwa 0,05 bis 5 mg/kg Körpergewicht an Dihydropyridin pro Tag zur Erzielung wirksamer Ergebnisse zu verabreichen, und bei oraler Applikation beträgt die Dosierung etwa 0,05 bis 20 mg/kg vorzugsweise 0,5 bis 5 mg/kg Körpergewicht pro Tag.

Trotzdem kann es gegebenenfalls enorderlich sein, von den genannten Mengen abzuweichen, und zwar in Abhängigkeit vom Körpergewicht bzw. der Art des Applikationsweges, aber auch aufgrund der Tierart und dem individuellen Verhalten gegenüber dem Medikament bzw. dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation grösserer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäss gelten hierbei auch die obigen Ausführungen.

Im Vergleich zu einer Kapsel ergibt die Applikation der Tropflösung eine schnellere Wirkstoffresorption und überraschenderweise höhere Blutspiegelwerte. Solche Tropfenformulierungen können auch bewusstlosen Patienten bei einem Vasospasmus nach Subarachnoidalblutung leicht per Schlundsonde ohne Komplikationen eingegeben werden.

Die schnellere Wirkstoffresorption und der höhere Blutspiegelwert sei am Beispiel Nimodipin anhand folgender Versuche erläutert:

6 freiwillige Testpersonen nehmen jeweils 60 mg Nimodipin mit 200 ml Wasser oral ein. Nach bestimmten Zeiträumen nach Einnahme werden die Blutspiegelwerte durch GLC ermittelt.

Nach Einnahme der erfindungsgemässen Zubereitung gemäss Beispiel 1 beträgt die höchste Blutplasmakonzentration von 115,5±78,3 µg/l nach 15 Minuten. Nach 24 Stunden liegt der entsprechende Wert unter der Nachweisgrenze von 2 µg/l. Der mittlere Flächenwert (AUC) der Kurve 1 beträgt 107,6±51,4 h.µg/l. Der mittlere Wert für die Bioverfügbarkeit wird berechnet zu 8,6+4,6%.

Die Fig. 1 zeigt den genauen Kurvenverlauf für die Lösung (-+-) und als Vergleich den der Kapsel (-0-).

Die zum Vergleich eingenommene Nimodipin-Kapsel liefert als Wert für die höchste Blutplasma-Konzentration 60,8±48,0 µg/l nach 30 Minuten. Nach 24 Stunden wiederum liegt der entsprechende Wert unter der Nachweisgrenze. Der mittlere Wert für die Bioverfügbarkeit wird errechnet zu 8,8±4,4%. Der mittlere Flächenwert (AUC) der Kurve 1 beträgt 91,6±35,8 h.µg/l.

Aus der Kurve sowie aus den Tabellenwerten kann der Wirkungsunterschied zwischen der erfindungsgemässen Flüssigzubereitung und der entsprechenden Kapsel leicht erkannt werden. Nach 15 Minuten beträgt die Blutkonzentration bei Einnahme der Kapsel 16,6±12,4 µg/l, während der entsprechende Wert der Flüssigkeitszubereitung 115,5±78,3 µg/l beträgt.

Tabelle 1

Blutkonzentration bei 6 Testpersonen nach oraler Einnahme von Flüssigzubereitung enthaltend 60 mg Nimodipin

| | Zeit nach Einnahme (h) | | | | | | | | | | AUC$_{0-\infty}$ (h. µg/l) | Bioverfüg-barkeit % |
|---|---|---|---|---|---|---|---|---|---|---|---|---|
| | 0,25 | 0,5 | 0,75 | 1,0 | 1,5 | 2,0 | 3,0 | 5,0 | 8,0 | 24,0 | | |
| 1 | 20 | 36 | 22 | 14 | 10 | 7 | 2 | <2 | <2 | <2 | 37,6 | 3,6 |
| 2 | 244 | 106 | 57 | 37 | 18 | 12 | 7 | <2 | <2 | <2 | 150,1 | 8,6 |
| 3 | 157 | 66 | 43 | 29 | 14 | 11 | 9 | <2 | <2 | <2 | 142,0 | 16,1 |
| 4 | 75 | 28 | 20 | 13 | 10 | 7 | 5 | <2 | <2 | <2 | 63,2 | 6,4 |
| 5 | 93 | 107 | 59 | 46 | 24 | 19 | 11 | 7 | <2 | <2 | 162,7 | 8,5 |
| 6 | 80 | 55 | 36 | 22 | 17 | 11 | 9 | 3 | <2 | <2 | 89,7 | – |
| $\bar{x}$ | 115,5 | 66,3 | 39,5 | 26,8 | 15,5 | 11,2 | 7,2 | 2,3 | <2 | <2 | 107,6 | 8,6 |
| ±s.d. | 78,3 | 33,9 | 16,7 | 13,1 | 5,4 | 4,4 | 3,3 | 2,4 | – | – | 51,4 | 4,6 |

Tabelle 2

Blutkonzentration bei 6 Testpersonen nach oraler Einnahme von Kapsel enthaltend 60 mg Nimodipin (Vergleich)

| | Zeit nach Einnahme (h) | | | | | | | | | | | AUC$_{0-\infty}$ (h.µg/l) | Bioverfüg-barkeit % |
|---|---|---|---|---|---|---|---|---|---|---|---|---|---|
| | 0,25 | 0,5 | 0,75 | 1,0 | 1,5 | 2,0 | 3,0 | 4,0 | 5,0 | 8,0 | 24,0 | | |
| 1 | 2 | 27 | 48 | 29 | 27 | 13 | 9 | 5 | 3 | 3 | 2 | 113,0 | 7,5 |
| 2 | 25 | 25 | 83 | 53 | 22 | 14 | – | 4 | <2 | <2 | <2 | 89,2 | 12,7 |
| 3 | 35 | 134 | 36 | 19 | 10 | 8 | 3 | 2 | <2 | <2 | <2 | 76,5 | 8,6 |
| 4 | 19 | 41 | 31 | 22 | 19 | 10 | 4 | 2 | <2 | <2 | <2 | 54,9 | 5,5 |
| 5 | 12 | 109 | 78 | 60 | 34 | 24 | 11 | 7 | 6 | 3 | <2 | 151,8 | 15,0 |
| 6 | 6 | 29 | 34 | 26 | 19 | 12 | 6 | 9 | 3 | – | <2 | 64,2 | 3,3 |
| $\bar{x}$ | 16,6 | 60,8 | 51,5 | 34,6 | 21,6 | 13,3 | 6,7 | 4,7 | 2,5 | 1,1 | <2 | 91,6 | 8,8 |
| ±s.d. | 12,4 | 48,0 | 23,1 | 17,4 | 7,9 | 5,6 | 3,5 | 2,6 | 2,1 | 1,3 | – | 35,8 | 4,4 |

**Patentansprüche**

1. Flüssigzubereitungen von Nimodipin, dadurch gekennzeichnet, daß sie aus Nimodipin in Mengen von 1 - 5 Gewichtsprozent bezogen auf 100 Gewichtsteile einer Lösung bestehend aus 30 bis 50 Gewichtsprozent des Lösungsvermittlers Glycerin-Polyethylenglykoloxystearat mit ca. 35 mol Ethylenoxid oder Glycerin-Polyethylenglykoloxystearat mit ca. 45 mol Ethylenoxid und 70 bis 50 Gewichtsprozent Ethanol und gegebenenfalls weiteren Hilfsstoffen bestehen.

2. Verfahren zur Herstellung von Flüssigzubereitungen von Nimodipin, dadurch gekennzeichnet, daß man den Wirkstoff in Mengen von 1 - 5 Gewichtsprozent bezogen auf 100 Gewichtsteile der fertigen Lösung gegebenenfalls unter Erwärmung mit 30 bis 50 Gewichtsprozent des Lösungsvermittlers Glycerin-Polyethylenglykoloxystearat mit ca. 35 mol Ethylenoxid oder Glycerin-Polyethylenglykoloxystearat mit ca. 45 % mol Ethylenoxid auflöst und dann 70 bis 50 Gewichtsprozent Ethanol und gegebenenfalls übrige Hilfsstoffe zugibt.

**Claims**

1. Liquid formulations of nimodipine, characterised in that they comprise nimodipine in amounts of 1 - 5 per cent by weight based on 100 parts by weight of a solution consisting of 30 to 50 per cent by weight of the solubilising agent glycerol polyethylene glycol oxystearate containing about 35 mol of ethylene oxide or glycerol polyethylene glycol oxystearate containing about 45 mol of ethylene oxide and 70 to 50 per cent by weight of ethanol and, if appropriate, further auxiliaries.

2. Process for the preparation of liquid formulations of nimodipine, characterised in that the active compound is dissolved in amounts of 1 - 5 per cent by weight, based on 100 parts by weight of the finished solution, if appropriate with heating, with 30 to 50 per cent by weight of the solubilising agent glycerol polyethylene glycol oxystearate containing about 35 mol of ethylene oxide or glycerol polyethylene glycol oxystearate containing about 45 mol of ethylene oxide, and then 70 to 50 per cent by weight of ethanol and, if appropriate, other auxiliaries are added.

**Revendications**

1. Préparations liquides de nimodipine, caractérisées en ce qu'elles se composent de nimodipine en des quantités de 1 à 5% en poids par rapport à 100 parties en poids d'une solution constituée de 30 à 50 % en poids du promoteur de dissolution oxystéarate de glycérine-polyéthylèneglycol avec environ 35 moles d'oxyde d'éthylène ou oxystéarate de glycérinepolyéthylèneglycol avec environ 45 moles d'oxyde d'éthylène, et 70 à 50% en poids d'éthanol et éventuellement d'autres substances auxiliaires.

2. Procédé de fabrication de préparations liquides de nimodipine, caractérisé en ce qu'on dissout la matière active en des quantités de 1 à 5% en poids, pour 100 parties en poids de la solution terminée, éventuellement avec chauffage, avec 30 à 50% en poids du promoteur de dissolution oxystéarate de glycérine-polyéthylèneglycol à environ 35 moles d'oxyde d'éthylène ou oxystéarate de glycérine-polyéthylèneglycol à environ 45 moles d'oxyde d'éthylène et qu'on ajoute ensuite 70 à 50% en poids d'éthanol et éventuellement d'autres substances auxiliaires.